# EUROPEAN PATENT APPLICATION

(11) **EP 4 481 643 A2**
(43) Date of publication of application: **25.12.2024**
(21) Application number: 24198720.5
(22) Date of filing: 03.11.2015
(51) Int. Cl.: G06Q 10/00

(54) **METHOD AND PLATFORM/SYSTEM FOR CREATING A WEB-BASED FORM THAT INCORPORATES AN EMBEDDED KNOWLEDGE BASE, WHEREIN THE FORM PROVIDES AUTOMATIC FEEDBACK TO A USER DURING AND FOLLOWING COMPLETION OF THE FORM**

(30) Priority: 03.11.2014 US 201462074130 P
(62) Divisional of application: 15857892.2
(71) Applicant: Automated Clinical Guidelines, LLC, St. Augustine, FL 32086 (US); Dew, Douglas, K., Sr., Palm Coast, FL 32137 (US); Halpern, Steven, J., Ormond Beach, FL 32174 (US); Engler, Hibbard, Willow Glen, CA 95126 (US); Steward, Duane, Bryan, TX 77808 (US); Dew, Douglas, K., Jr., Palm Coast, FL 32137 (US); Dew, Dylan, R., Palm Coast, FL 32137 (US); Stratton, Sandie, A., Hasting, FL 32145 (US)
(72) Inventor: DEW, Douglas, K., Sr., Palm Coast, FL (US); HALPERN, Steven, J, Ormond Beach, FL 32174 (US); ENGLER, Hibbard, Willow Glen, CA 95126 (US); STEWARD, Duane, Bryan, TX 77808 (US); DEW, Douglas, K., Jr., Palm Coast, FL 32137 (US); DEW, Dylan, R., Palm Coast, FL 32137 (US); STRATTON, Sandie, A., Hasting, FL 32145 (US)
(74) Representative: Page White Farrer

(57) **Abstract**

There is disclosed a system for guiding a user's encounter with a patient and for determining a patient medical condition.

## Description

### CROSS REFERENCE TO RELATED APPLICATIONS

This patent application claims the benefit under Section 119(e) of the provisional patent application assigned Application No. 62/074,130 and filed on November 3, 2014, which is incorporated herein in its entirety.

### FIELD OF THE INVENTION

This patent application relates to a method and system for creating a web-bawd form that incorporates an embedded knowledge base.

### BACKGROUND OF THE INVENTION

Proper documentation of medical imaging findings, including anatomical location, joint position, arthritis location with severity, fracture patterns, bone changes, changes from joint arthroplasty, and ICD-9/10 coding, are a vital aspect of a physician's imaging report. Radiographic data is recorded as an x-ray report for insurance reimbursement and for the patient's medical record. The important outcome data likely ends up in the clinical "black hole" of an individual patient's chart.

Electronic medical records (EMRs) are now finding their way into private practice but these systems offer little in the form of software suites for documenting imaging findings and for generating standard and therefore searchable imaging documentation. While radiological software is available for certain templated studies, there is little in the way of imaging electronic documentation other than templated "pick-lists" for common findings. The currently available systems suffer from one or more of the following shortcomings: templates and "pick-lists" range from too simple to complex, creating confusion among the users; minimal use of standard nomenclature; hardware, software and support is expensive; physicians lack time to customize the system; physicians lack time to learn a new program, especially a complex one; screens are too "busy" and too many windows open at any given time; minimal use of published clinical guidelines.

ICD-9, formally referred to as The International Classification of Diseases, Ninth Revision, was the official system of assigning codes to diagnoses and procedures associated with hospital utilization in the United States. This code was replaced by ICD-10 on October 1, 2015.

Implementation of the new coding system will not be easy and is expected to take several years for full implementation and full specifity.

Generally, the code numbers indicate the category of disease, the cause or origin of the disease or condition, the body part affected and the severity of the condition. The new code requires the installation of new software and medical practices must provide training for physicians, staff members, and administrators. They will also need to develop new payor rules, new practice policies and guidelines, and update paperwork and forms. For convenience, practices may also create "crosswalks" that will convert their most frequently used ICD-9-CM codes to the ICD-10-CM equivalents. In the further there will need to be a conversion to ICD-11 and others as well.

The present invention teaches a method and platform/system to make this transition easier for all involved with the ICD -10 code system as well as providing other advantages for creating medical records and for diagnosing medical conditions. It is modular, validated, easily updated and can incorporate large volumes of data and text very quickly

Information related to medical conditions is complex and growing every day as new information is added to the growing medical library. Commonly accepted practices and procedures that generally result in a successful patient outcome cannot be known by all specialists in afield. Additionally, the medical field includes third party payors who will reimburse medical expenses only if certain protocols and guidelines are satisfied. Thus the medical professional must endeavor to know and then follow these protocols and guidelines to achieve a successful outcome for the patient and to ensure compliance and thereby compensation from these third party payors.

Given the complexity of the field, the paperwork demands to memorialize the patient encounters, test results, clinical examinations, etc. can be overwhelming.

### BRIEF DESCRIPTION OF THE FIGURES

The forgoing and other features of the present invention will be apparent to one skilled in the art to which the present invention relates upon consideration of the description of the invention with reference to the accompanying drawings. The use of the same reference numeral in the various figures refers to the same element.
Figure 1 is an excerpt of a document as created by a subject matter expert for the failure to thrive syndrome.
Figure 2 is an excerpt of the document of Figure 1 converted to a rich text format.
Figure 3 is an excerpt of an scr document based on the document of Figure 2.
Figure 4 is an excerpt of a T-spec document based on the document of Figure 3.
Figure 5 is a software flowchart illustrating the steps associated with creating a web-based form according to the teachings of the present invention.
Figure 6 is a block diagram of a processor and associated components for implementing the teachings of the present invention.

### DETAILED DESCRIPTION OF THE INVENTION

Before describing in detail the particular method and platform/system for creating a web-based form incorporating a knowledge base, it should be observed that the present invention resides primarily in a novel and non-obvious combination of elements and method steps. So as not to obscure the disclosure with details that will be readily apparent to those skilled in the art, certain conventional elements and steps have been presented with lesser detail, while the drawings and the specification describe in greater detail other elements and steps pertinent to understanding the inventions.

The presented embodiments are not intended to define limits as to the structures, elements or methods of the inventions, but only to provide exemplary constructions. The embodiments are permissive rather than mandatory and illustrative rather than exhaustive.

Certain of the terms, system descriptions and features of the invention described herein are also described in co-owned US patent number 7,962,348, which is incorporated in its entirety herein.

Form-based input of information is an enabling technology that permits widespread distribution of form-based applications across client platforms, such as a conventional content browser. In the context of Web-based forms, a markup language defined interface can form the principal conduit through which end users can interact with backend application logic. Often configured in the form of a Web page, the interface can be provided to the content browser by a content server, and can take the form either of a pre-defined static page, or a dynamically generated page. Form input fields can be positioned within the interface through which user input can be accepted and posted to the backend application logic for further processing.

The present invention comprises an interactive method, platform and system for directing, integrating, documenting, and tracking steps taken by a medical provider during the patient encounter. A medical professional's actions are directed or guided by prescriptive protocols, guidelines, payor requirements, etc. comprising prompts for information that together with the responses create a path through a decision tree or decision engine. The tree comprises a plurality of nodes and a plurality of branches interconnecting the nodes. Each node is associated with an answer to a medical inquiry (clinical examination, medical test result, etc.) and each branch represents a conditional dependency or inquiry between two nodes. A graphical representation of the branches and nodes would appear as a tree. The decision tree can be easily updated as more current medical information becomes available. The invention thus offers optimal patient care (i.e., providing commonly accepted testing and treatment protocols), enters the necessary codes for each test, examination, etc. for the patient's specific medical condition, and documents to details of each patient encounter.

One aspect of the present invention relates to an all-encompassing method and platform (e.g., hardware components comprising a system and controlled by software) for creating and developing browser-accessible forms. The form structure allows for the efficient structured organization of a large amount of data and text The forms operate in conjunction with a knowledge base (e.g., a decision engine) that, under control of a processor or server, guides a user, step-by-step, as he/she completes the form by entering the requested information.

According to another aspect of the invention, the knowledge base directs the user's inputs by presenting the right prompts at the right time, in particular based on the user's prior informational inputs and prior responses to prior prompts. Once the form has been completed, the system presents the entered information in a desired format.

The forms can be accessed and completed using conventional web browser software. During a structured work flow process as the user responds to each question or prompt, the knowledge-based platform provides the user with the next appropriate prompt or question. This process ensures that the form is completed correctly and completely, i.e., with all necessary and sufficient elements as requested by the form.

For example, a diagnosis based on specific symptoms, findings and other patient medical information (e.g., findings or results from one or more of medical tests, medical procedures, clinical examinations and from other sources that provide patient medical information that is relevant to determining a diagnosis and developing a treatment plan) is more likely to be correct only if all the required elements related to that diagnosis have been entered and entered correctly. Also, compliance with payor coverage rules is assured only if all elements related to payor coverage requirements have been accurately and completely entered (e.g., have all the required medical protocols and guidelines been followed) and the correct diagnostic or treatment codes assigned. In one embodiment these ICD codes are automatically identified and recorded in real time as the user completes the form.

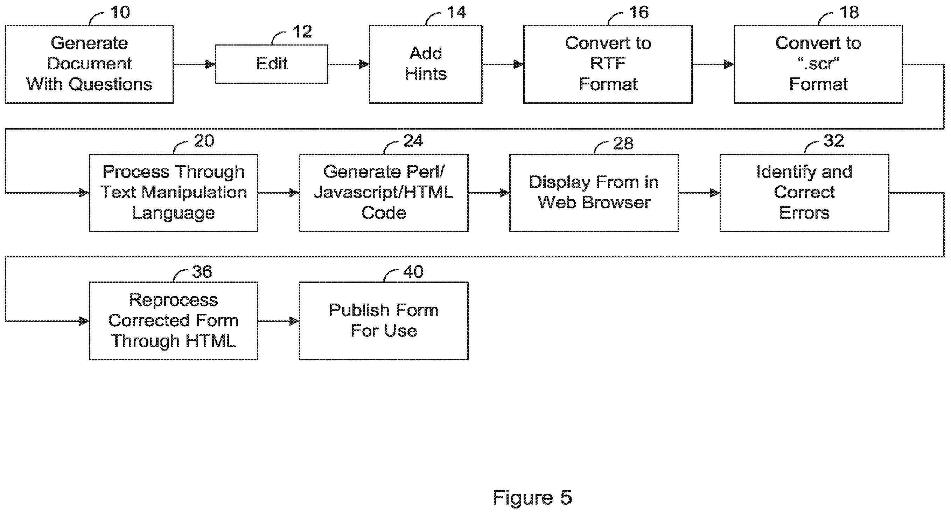

In one aspect, the platform or system serves as a guide for a user's examination of a patient and for generating a transcript of that examination. One example of such a system is illustrated in Figure 5 and described below. Generally, the system, according to one embodiment of the invention, comprises a processor or server for controlling an output display device or screen and an input user interface through which the user enters information for analysis by the processor or server.

According to one embodiment, the processor or server is programmed to output to the display device: (a) a screen requesting patient reference information, wherein the patient reference information may exclude a patient name (but may include a unique patient assigned number, for example) and further requesting patient medical history information; (b) a screen requesting vital signs information; (c) a screen presenting a plurality of symptoms in text form, from which the user can identify one or more observed symptoms (that is observed as the user conducts a clinical examines the patient) as selected from among the presented symptoms or the user can indicate that no symptoms are observed; (d) in response to the user identifying one or more observed symptoms, the processor controls the screen output to displays a plurality of anatomical regions for examination. In one embodiment the plurality of anatomical regions are presented in text form and the user selects an anatomical region based on the one or more observed symptoms. This is done in a graphical user interface via relevant icons or pictures instead of a drop down menu.

The processor is further programmed to: (e) present on the display device anatomical subregions within the selected anatomical region, the anatomical subregions are presented in text form in one embodiment or in illustration form in another embodiment. The user selects an anatomical subregion based on the one or more observed symptoms based on relevant icons as well.

The processor is further programmed to; (f) display one or more screens that show a rendering of the selected anatomical subregion and possible findings associated with a condition of the patient as related to the selected anatomical subregion. The renderings depict normal and abnormal conditions of the selected anatomical subregion to assist the user with selecting one or more findings. The processor presents more-detailed renderings (in a drill-down fashion) responsive to the user rolling a cursor or touch screen, for example, over selected regions of the renderings.

The processor is further programmed to: (g) as the user selects findings, additional renderings are presented related to the selected findings (this is accomplished in a drill-down fashion as the user selects findings more detailed renderings are presented; (h) wherein one or more of the screens include an icon for allowing the user to switch from the physical examination to an examination of a radiographic image.

The processor receives user selections from the user interface and generates a patient transcript (e.g., patient medical conclusion) responsive to information entered into the screens during the steps (a) through (h). The transcript may be printed on a printer.

Other inputs to the system include entry by gesture, motion (accelerometer), text entry, key strokes and recorded video, audio and scans.

As is known by those skilled in the art, the processor and its attendant display device and input user interface can be an element of a desktop or laptop computer and thus the system /platform of the invention can execute on such a desktop or laptop computer. But with the burgeoning inclusion of processors and displays in many electronic devices today, the system/platform can also execute on a tablet computer, smart phone, notebook computer, Google Glass ^{™} device or any other device having the requisite processing power and display capabilities. Such devices can be physically tethered to a source of power and/or data or can operate wirelessly (e.g., mobile devices).

One application for the teachings of the present invention is the health care industry. Prior art electronic medical records (EMRSs) are a systematic collection, in digital form, of health information about an individual patient. The EMRs are database driven and serve as a repository for patient health information. Unlike the present invention, the EMRs have multiple shortcomings including lacking prompts to lead the physician through the examination, diagnosis and treatments processes and no element for ensuring that the data is entered during collection of the information in such a way that omissions and incomplete entries are prevented.

As discussed above, health care providers are now required to utilize a new classification system for coding a patient's health conditions and medical procedures. The new system, referred to as ICD-10, is intended to overcome the many administrative issues associated with medical care and payment by payors for that medical care. With the 69,000 codes in ICD-10 and about 14,000 codes in ICD-9, the former code structure is significantly more granular, thereby improving over the latter code structure by providing more information and more precise information, by providing better support for care management, quality measurement and analytics, and by offering improvements in the representation of risks and severity of medical conditions.

To accurately code a fracture according to ICD -10, codes related to the site, laterality, type and location of the fracture must be entered. For an angioplasty condition, ICD-9 lists a single code whereas ICD-10 lists 854 codes.

The challenges associated with accurately coding according to structured medical knowledge, clinical guidelines and ICD-10 are numerous (too many codes make it difficult to identify the correct one, lost time searching the codes, omissions and errors, missing documentation, missing examination elements, selection of a code that is not supported by the medical record documentation, etc.) and exacerbated by the large number of ICD-10 codes. Furthermore, the codes of ICD-9 do not all map one-to-one to ICD-10 codes. There are one-to-many, many-to-one and many-to-many type relationships between the two code sets. This adds to the difficulty posed to those familiar and habituated with the predecessor as the newer code replaces the older code. The data and choices are even more complicated than ICD-10 coding when the user is performing an examination, aggregating clinical data, and selecting the correct diagnostic choices and clinical treatment pathways for the patient's care.

A software component (e.g., an embedded or stored knowledge base as controlled by the processor or server) of the invention builds decision trees on a platform based on a subject matter expert's input and logical organization of the embedded knowledge. The platform operates like a GPS system to guide the user with a series of choices or questions (prompts or prompt fields) that traverse the decision (knowledge) tree created by the software as the user enters response to the prompts. This process creates a path through the decision tree. There are error checks to coding, physical exam, x-ray/imaging, lab results, and symptoms, etc. that in effect lead the user down a checklist (i.e., a path through the decision tree) to an answer or answers (diagnosis and/or treatment plan). This system incorporates web-based analytics track the interaction in real time and via location for improved population health as well as disease and injury registries.

As the path is followed, the entered information is cleaned, normalized - both in terms of claims (coding data) and clinical data - to place in disease, injury, or medical registries, as well as population analytics, for later analysis and use in epidemiological studies. A dashboard is also provided for quality improvement with real time feedback to the user or an institution.

The inventors have thereby created, in essence, a decision tree to prompt and guide the user, based on user inputs, to a desired branch where the best answer will be found at the end of the branch or at the final "leaf." The leave is the output product of the web based structured form. It contains analytics based on the branches that are traversed by the user as well as location of the user among other collected data. The leaf contains text, structured data, coding, guidelines, and clinical data that matches claims data. As new information is or data added by a subject matter expert the system deposits this information at the base of the tree (the registry or knowledge base) and the knowledge tree grows a strong foundation by growing new "roots" based on this new information or data. In other words the system grows and "learns" from aggregating data from one or more sources in a normalized or standard fashion in the form of a dashboard or decision tree. The system creates data and analytics so the user or a combination of users learn from their interaction with the system to update any knowledge bases. For example, in medicine, the system learns as it is taught through the input of new or updated forms to improve medical knowledge and medical care based on the structured analytics of collected data.

The invention presents a learning healthcare system that can lead to new research in new fields and reaches new conclusions based on analysis and peer review of the entered information. These results can be fed back into the healthcare delivery process and embedded into the decision tree system platform for future use.

Forms associated with one aspect of the invention record individual data points for each presented/displayed check box or radio button. The use of these boxes and buttons capture relevant clinical information, and other patient medical information that is then recorded as structured data.

The present invention seamlessly embeds approved health care guidelines that are used by the physician during the examination, diagnosis and treatment processes; fully codes and documents for care delivery, coordination, and billing; produces searchable data that is fully uncoupled from all patient identifiers (thereby reducing risks to patient privacy and security); provides auditable documents for proving adherence to Local Coverage Determinations (LCDs) and National Coverage Determinations (NCDs), HCC's(Hierarchical Condition Categories) and eliminates high CERT (Comprehensive Error Rate Testing) error rates and thereby minimizes claim rejections.

The system of the present invention can dramatically reduce health care delivery costs (or costs associated with any industry that uses the invention) and substantially improves quality of care with resources focused on patient care, but not on administrative overhead and bureaucracy (e.g., reconciling accurate codes after the fact, i.e., after the patient encounter). The present invention uniquely integrates CMS (Centers for Medicare and Medicaid Services) coverage determinations and other payor rules, specialty society guidelines, government regulatory practices, ICD-10 codes, and error audits so that patients, physicians and payors can work from the same documentation and the same rules. The result is an improved level of soundness in the record with less expense. All of which will result in improved patient care.

The invention couples the clinical documentation data entry process with applicable guidelines, quality measures, coverage compliance, etc. from the point at which the data is entered, i.e., from the point of origin of the data. In so doing, and in so far as the data entry is as easy to perform as any alternative, compliance with applicable standards, codes, etc. is no longer considered a separate reporting effort, where that separate reporting effort requires the separate investment of time, energy, processes, expenses and burdens beyond the delivery of care to the patient. All of these attributes provide direct patient value. Any need for quality measurement, policy compliance, protocol adherence, etc., then becomes a matter of a data query (i.e., querying the medical record generated by the present system or platform), rather than an effort of data reporting.

According to another aspect, the invention comprises and also creates (as described further below) web-based forms that embody a knowledge base, the contents of which are hierarchically presented in a context-sensitive manner. As the form is completed (i.e., the data entered by the user during the patient encounter, for example), the inventive platform performs real-time error checking and data entry validation, and produces standardized reports that can be easily attached to electronic records, used as an input file for a database, or used in a health information exchange. As the data (i.e., patient medical information) is entered a path through the decision tree is created. The knowledge database is uniquely based on the International Classification of Diseases (ICD), is always updated as to version, and thus serves as a standard and primary data key for information exchanges to any existing EMR, disease registry, or payor system much like a SIM care found in one's portable phone.

The data-enterable forms as presented on the output display device include the following attributes:
Self-contained
Hard-coded
Printable
Importable
Electronically transferable
Standardized nomenclature

Elements of one or more of the forms may comprise:
Form description
Questions with candidate answers. The candidate answers may be presented as:
   Checkboxes
   Radio buttons
   Lists of candidate answers where answers are selected to enter data
Questions eliciting a numerical value as a response. (The user can enter a numerical value as a response or the question may include a list of candidate numerical values from which the user selects a value.)
Free-text entry fields (In an effort to make completion of the form as easy as possible, the use of free-text fields is minimized to the extent possible)
Required questions and optional questions
Hidden/expandable text. (That is, text that is hidden until the associated checkbox or radio button is checked or marked thereby causing the form to expand to display the hidden text. The use of hidden text makes the form easier to read as unnecessary information is not displayed unless and until required.)
Embedded logic to ensure that the form will be completed correctly and completely when the entered data is later converted to a form.
Form validation before it is deemed complete
Visual displays of correctly selected items
Visual displays of required but not selected items
A graphical user interface on anatomy, guidelines, and/or ICD coding
Real time notification when selecting specific questions
Cartoon images, animated images, photos and videos to assist the user in completing the form
Processing/navigation buttons (For example, log out, review transcript, return, generate PDF report, etc.)

The system or platform, using the internally structured form as a base, can programmatically expand the form into a natural language narrative or generate detailed/bulleted contents and reports.

The form is exchange-ready and normalized , that is, for health information exchanges, HL7 export and electronic messaging.

The encounter between a physician (also referred to herein as a user or a clinician) and a patient may be represented by four fundamental components: the context, the taking of clinical history, the collection of vital signs, and ultimately the physician examination. Although the form described herein is primarily focused on guiding the physician's examination component, the elements of the invention can be expanded to include and the same concepts similarly employed to assist the other three listed components of the physician encounter.

Note that outcomes from each of the four listed components and any findings or data gathered during each of the four components, may govern the path that is traversed through the decision tree during the physical examination. Thus the physical examination proceeds as the user, in conjunction with data collected from the patient, essentially traverses through the decision tree during the examination, where the path through the tree is developed as new patient medical information or data is entered and the expert knowledge base is consulted under control of the processor. For instance, details from the clinical history may eliminate some questions in the other three components, shape the substance of other three components, and spawn certain questions unique to the clinical history as presented to this point. Note further that the embedded or implicit algorithm that governs the tree-traversal details for the examination component is in part dependent on these four areas, among other aspects of the patient encounter.

When the present invention is used, there is no obtrusive process of data entry or distraction from the patient encounter or examination, and no disruption of workflow or the user's cognitive processes while attending the patient. In contrast, a prior art medical record entry processes require a cognitive interruption to translate from the natural flow of the patient encounter to ascertain the necessary and sufficient elements to record, perhaps with an unnatural device (e.g., haptic control or a spin of the stool toward a computer monitor to navigate a busy user interface, etc.).

The present invention blends transparently with the workflow associated with the patient encounter and reports the data captured without requiring an additional or a separate effort. Thus the invention stands in stark contrast to present separate efforts to extract patient status information, examine the patient, create a record of the examination findings, file reimbursement claims, retrieve comparative effectiveness data, and submit quality measures, etc.

Since the form is an active document that operates in conjunction with a background processor-based platform or knowledge base, it offers many attributes beyond a passive form (whether paper or digitally-based). Some of these attributes comprise:
Serves as a medical record complete with diagnostic codes entered as the entries are made
Useful for preauthorization requests
Provides evidence of justification for insurance coverage/payment
Contributes to required compliance log
Useful as a quality measurement tool
Provides defensible documentation for record audits
Browser platform independent
Operative in a stand-alone mode without Internet access
Operative in a stand-alone mode without use of a browser
Structured (i.e., reliable, consistent, quality assured, efficient)
Gathers evidence for later use in evidence-based medical practice
Creates a non-taxing burden in the care-delivery workflow
Provides Medicare compliance by the design of embedded inferences and logic in the knowledge base that directs the patient examination by presented prompts
Creates the required documentation and ensures the documentation complies with applicable rules and regulations
Provides easy and natural data entry
Transferable to mobile and other platforms
Provides easy touch screen data entry with minimal key strokes
Provides data entry with icons, check boxes and radio buttons (augmented by free text entry only as necessary). A key differentiator relative to the prior art as the prominent use of check boxes and radio buttons and minimal use of free text entries normalizes the collected data at the point of entry.
Offers visual cues as to compliance or failure to comply with data entry elements
Offers immediate validation as to correct and complete data entry
Provides central maintenance of form logic and constraints
Can be hyperlinked to Internet-published guidelines, quality measures, coverage determinations, protocols and care plans
HIPAA compliant
Reduces risks of non compliance
NCD/LCD sourced
Includes/embeds ICD coding within the physician's workflow, i.e., ICD coding is automatically entered as the physician conducts the clinical examination
Explicit and exhaustive in its content (i.e., few options are available in response to most questions)
Integrates CMS and specialty society guidelines, government regulatory practices, ICD, and error audits
Uniquely originates from CMS coverage determination knowledge base
Captures quality-measure data
Operative in conjunction with a web service
Expandable to include any medical specialty
Clinical documentation is normalized, i.e., conforms to norms that ensure a completely functional and definitive output, in real-time as the data is first captured and entered into the form
Qualified data entry. i.e., coupling of clinical data capture with a guideline, care plan or coverage determination to which the data is compliant at the point of entry, such that it meets those requirements, fulfills the embodied purpose and retains satisfaction by perpetual association
Creation of qualified data as it is entered in real-time
Utilized at the patient bedside and during any patient encounters
Couples patient generated data, clinical findings, examinations, labs, imaging outputs with immediate coverage determination, preauthorization, qualification, practice compliance by way of formal guidelines, protocols and policies (from professional associations, enterprise standards, regulatory bodies, payor declarations, advanced directives, and governing authorities)
Applicable to conditional authorization of medication, diagnostic test or therapeutic procedures
Tree traversal driven by anatomical, clinical, and radiographic observations
Constrained branching during tree traversal
Linked to other information sources: indications (prescription, surgery, consultation, admission), contraindications (prescriptions, medical procedures, hospitalization, exposures), policies, advanced directives, disclosures and access privileges
Centralized implementation delivered as a service (Internet API)
Incorporates error checking
Classification hierarchy is generated as the decision tree is traversed and branch selections are made
Easily transcribed to a report based on tree traversal
Permitted branching limits paths at each tree node (i.e., constrained branch traversal between branches)
Parsing of the tree traversal algorithm is balanced with minimization of clicks to complete traversal
Includes point & click on cartoons, icons, line drawings or diagrams where this is a more efficient data entry technique through a graphical user interface
Traversal of the tree is governed by embedded links
The logic tree embedded in the form includes:
   procedures
   diagnostic
   qualifying
   grading
   therapeutic
   palliative indicated
   treatments
   options
   conditional implications
   previous results
   among others
The logic tree embedded in the form includes standardized identifiers for
   HMS
   reimbursement
   prevention
   reportable diseases
   procedures
   quality measures
   incentives
   compliance
   among others
The logic tree embedded in the form includes
   compliant guidelines
   record of all requisites
   conducted actions
   satisfactions
   consequent expectations, approvals or permissions
Context sensitive
   restricted to necessary and sufficient
   restricted to branching-factor constraint, classification tree traversal path specific (i.e., order of sibling pathways from tree dependent upon context)
   metadata
      provider
      practice
      scenario
      acuity
      pathologic phase
      epidemiologic features
         hereditary
         communicable
         highly infectious
         transmission mode
      recurrence
      scale/class/index value (e.g., Rankin Scale level)
The logic tree embedded in the form includes criteria related to:
   negative for requisites
   prior and posterior probability attributes of findings, tests and procedures
The logic tree embedded in the form includes exclusion criteria:
   elimination for positive findings
   prior and posterior probability attributes of findings, tests and procedures
Consolidates working code such that wrapping technologies (e.g., Google Translator and Google analytics) can be employed to extend technical capabilities
Explicates co-morbidities
Identifies risk assessment contributors
Provides for past-history (in both long and short versions)
Offers an easy-to-use user interface with immediate visual non-verbal feedback in the form of highlighting
Explicit use of the motivating content (coverage determination, guideline, etc.) to govern tree traversal and generate output to a desired level of detail
Abandons constraint of a single screen for an unlimited vertically scrollable form
Back peddling through the form is minimized or absent
Determines and quantifies risk
Inherent audit trail provided by a transcript based on the tree traversal
Encapsulated and embedded constraints, relationships and compliance criteria propagate by implication and semantics to generate both:
   clinical record
   bulleted audit statement
Provides justification for
   guideline election
   prequalification
   coverage determination
   compliance attestation
   report generation
Generates structured content, message or standardized input tailored for
   user analytics such as time, date, location and pathway analytics
   outcomes analysis
   results measurement
   performance monitoring
   compliance documentation
   population health management
   interactive decision support
Voice recognition-based data entry (similar to constrained input solicited by phone or a limited auditory cue)
Maintained by central updates without disruption of service
Extendible by transparent expansion of central services
Secured and protected by centralized account security and privacy technology including unique user identifiers, authentication and authorization
Networked with external services and support for content, policy, guidelines, records and status monitoring
Tailored to site-specific content or customizations for practitioner, practice, network, or geographic region
Generates data and information necessary to populate dashboards, data warehouses, utility reports, attestation, validation and verification
Provides potential for aggregation of alert incidence and nature
Generates feedback regarding compliance deviations with extended information regarding the scope, frequency distribution and context of deviation and exception, along with factors potentially accounting for variation and repetition risk
Includes formal capture of metadata and conceptual semantic ontology sufficient to ensure adequate support for arbitrary machine query without human mediation necessary
Captures clinical information structured with basic formal ontology
Web based User (provider) analytics such as time, date, location, and treatment pathway & outcome analytics
Web based payor analytics such as time, date, location, and treatment pathway & outcome analytics
Web based patient analytics such as time, date, location, and treatment pathway & outcome analyticssufficient to retain the complete semantic meaning of the data such that using the data in the future (population health, outcomes analysis, performance measures, accountable care, process improvement and other purposes mentioned afore, etc.) will not require human mediation
Provides for risk determination and quantification
Tracks progress of specific problem, disease, patient, practitioner, practice, network or region with no loss of generalization (e.g., zip code, altitude, environmental condition, economic strata, population density, race, religion, etc.)
Extracts unanticipated relationships between data elements, adverse events, bio-surveillance markers, epidemiologic indicators, care plan compliance, prognostic reckoning, etc. in real time.
Achieves the former with ability to breakdown by age, socioeconomic factors, temporal features, risk categories, genotype, phenotype, incident frequency, etc.
Prescribes the analytics and analytic variation required for each branch of the classification hierarchy decision tree for each scenario and the process of its traversal as they vary across nodes and branches.
Prescribes the implications of workflow on the structure of the classification hierarchy decision tree for each scenario, the process of its traversal and ramifications for effective timing in whole or in part.
Generates alerts regarding discrepancy between actuarial measurements and projected indicator or policy thresholds

When the user believes that all form elements and questions have been completely addressed, he/she clicks a "Review Transcript" button on the form. At this step the validity of the data entered is performed if validation was not embedded into the logical structure of the tree-traversal or individual questions and available answers. Omissions or errors in the completed form cause the form to be redisplayed with the omitted or error entry indicated until all form elements comply.

According to the prior art, to create a web-based form, such as one embodying the concepts of the present invention, a subject matter expert creates form content. That document is given to a programmer who uses text manipulation programming languages to create code in the Perl, HTML, and/or JavaScript languages for displaying and completing the form on a web browser. One aspect of the present invention automates this process to create the form by structured logic.

To create the form (and its background platform) a subject matter expert in a field pertinent to the subject matter of the form uses his/her knowledge and expertise to create a document posing questions that prompt the user for information. In some case the subject matter expert also lists possible responses from which the user of the form can select one response. This document is typically created using conventional word processing software such as Microsoft Word using unique logic symbols.

The document content robustly conforms to applicable guidelines, protocols, coverage determinations, professional specialty guidelines, written standards, etc. that represent the standard of care for the patient. The document created by the subject matter expert may further include:
Questions in the format as they will appear on the form
Selectable answers in the format as they will appear on the form
Checkboxes with candidate answers
Radio buttons with candidate answers
Lists of possible values in response to questions
Text fields (i.e., fields for entering information in free text)
Hidden text fields
Informational text in the format it will appear on the form
Mandatory and voluntary questions
Pop-up warnings
Form validation aspects
Output requirements

For example, if the form is for use during a physician's clinical evaluation of a patient, the questions prompt the physician to record patient symptoms and to check the patient's body for any signs of disease.

An editor then reviews the expert's document for grammatical errors, makes corrections as required, adds hints or templates (coding) to the document, and generates a rich text format (RTF) document. The conversion to an RTF format removes the special characters and formatting indicia that may have been present in the document created by the subject matter expert.

This process also preserves as much of the verbiage as possible from the original source document such that the resulting RTF document will be more explicitly conforming after findings, etc., are translated by the invention into a natural language narrative output. The narrative output is recognizably similar to the source that it disarms any contention of non-compliance or inadequacy.

The hints provide direction for a text manipulation programming language, which will eventually process the document as described below, with the information necessary to generate code that will create a web-based form deployed for access by a web browser.

Typical hints may relate to:
Questions to be displayed in a checkbox format that permit the user to select multiple responses from among candidate responses
Questions to be displayed in a radio button format that permit the user to select only a single response from among candidate responses
Questions that require the user to enter a value, a list of acceptable candidate values may be provided or the user may be required to enter a value
Text fields prompting the user to enter free text
Hidden/Expandable text fields, i.e., the text is hidden until a specific checkbox or radio button is checked, at which time the form expands to display the hidden text under the checked checkbox or radio button
Question requirements
   Is the user required to answer the question?
   Is the user required to answer the question dependent on his/her answer to one or more other questions?
Pop-up warnings
   When the user omits a required action or performs an inappropriate or incorrect action a pop-up warning is displayed that explains the nature of the problem
Form layout and structure
   Indentations, spacing, background color
   Waming/note locations and display colors
Report output requirements, including:
   Natural language format
   Outline/bulleted format
   Database compatible format
Form validation process, which is accomplished with JavaScript code

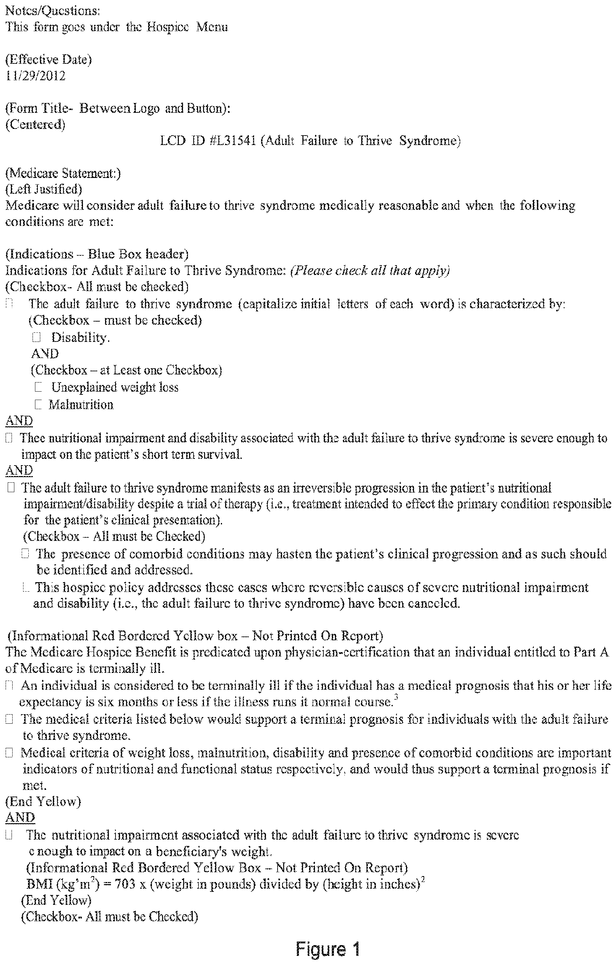

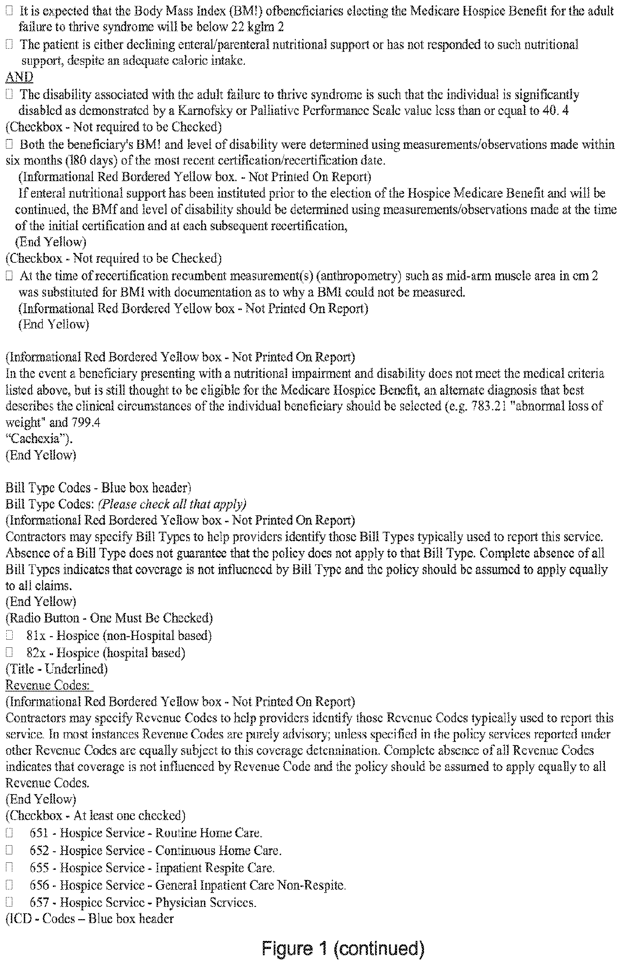

Figure 1 is an excerpt from such a document as created by a subject matter expert. The web-based form derived from the Figure 1 document is for use by a physician in determining if the patient exhibits an adult failure to thrive syndrome (an ailment characterized by a gradual decline in the health of an older adult without an immediate explanation).

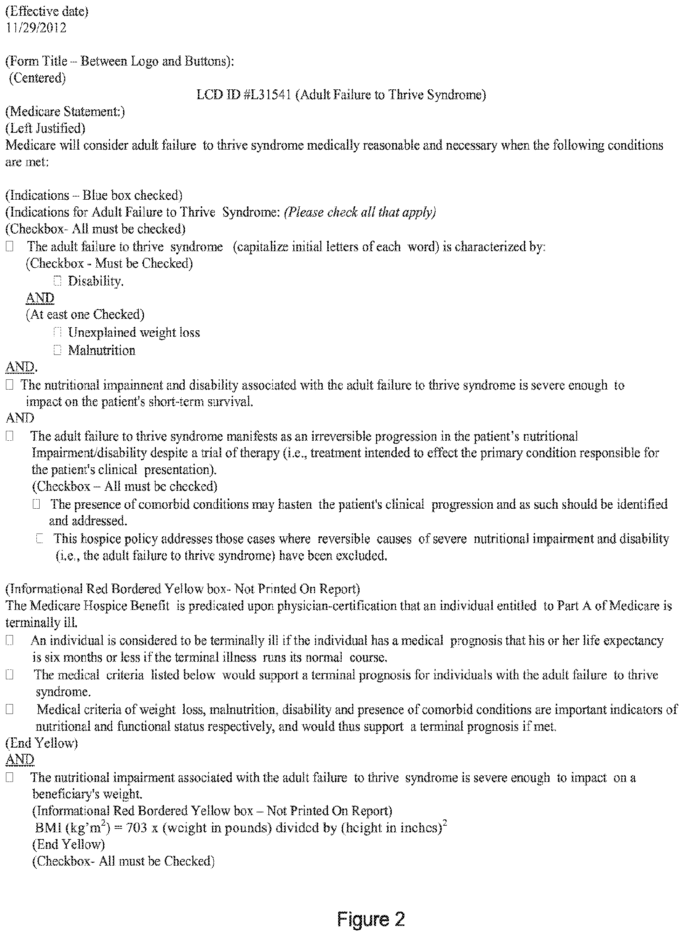

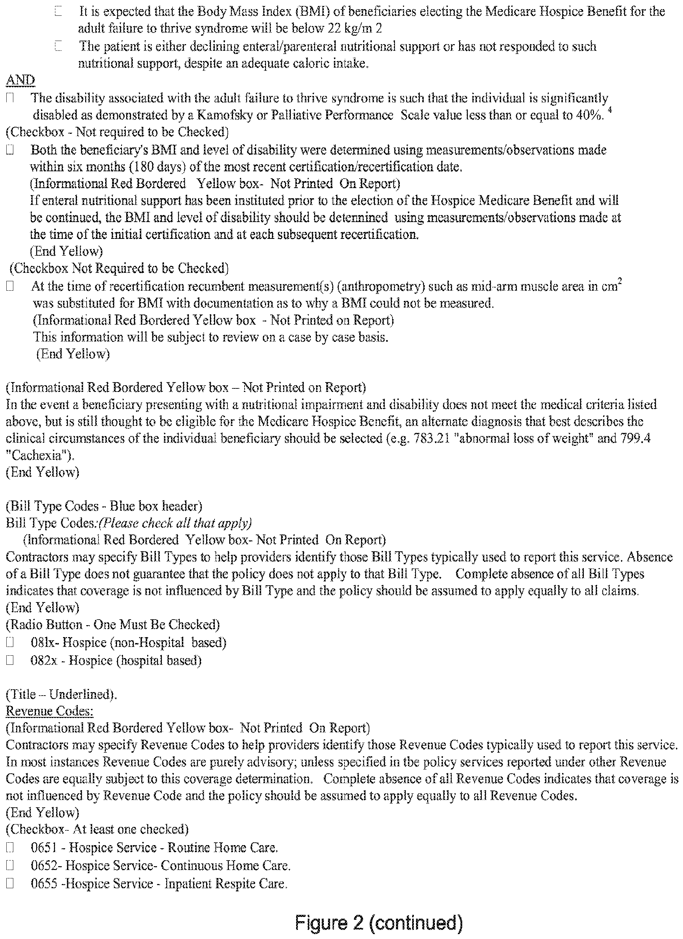

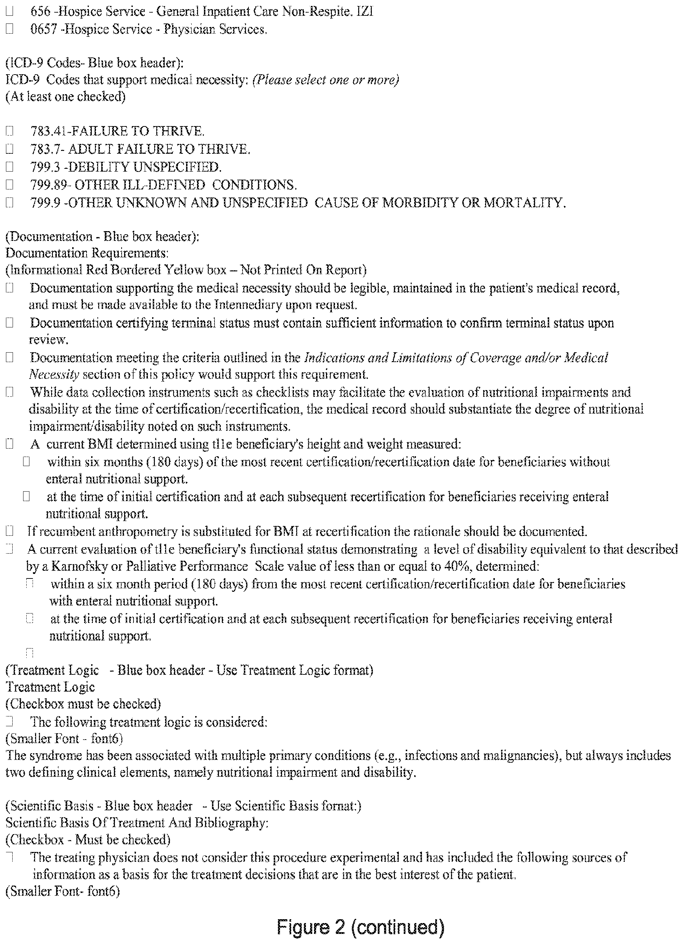

The word processing document of Figure 1 includes special characters and formatting commands. These are removed when the Figure 1 document is converted to a rich text format document as depicted in Figure 2.

The rich text format document with hints/templates is converted to an .scr file, i.e., a text file, by a SNOBOL program. Going forward according to the process of the invention, the scr text file is easier to parse. The SNOBOL program also creates a T-spec (specification) file. The SNOBOL program is a text manipulation language that has been programmed to receive as inputs the hints/template document to produce an appropriate output (e.g., a series of check boxes are generated as an output responsive to the check box hint).

SNOBOL uses pattern matching and coding to generate a document in a T-spec language invented by the inventors. The T-spec language is a "name, value pair" language similar to xml, but more concise, readable, and writeable without requiring special editing software. The T-spec language simplifies creation of the final form. Thus the SNOBOL program and the T-spec document allow a computer to create the web-based form without intervention by a human programmer.

One inventive aspect of the present invention is the generation of the T-spec language from the source document written by the subject matter expert. The SNOBOL code to do this adapts (with the help of developers) to the changes and slight differences one finds in human written documentation. Also, the SNOBOL program is intelligent enough in some cases to redo the output to fit a standard output. These intelligent modifications may include punctuation, capitalization of first words that are not acronyms, or rephrasing error messages, instructions for the user, etc.

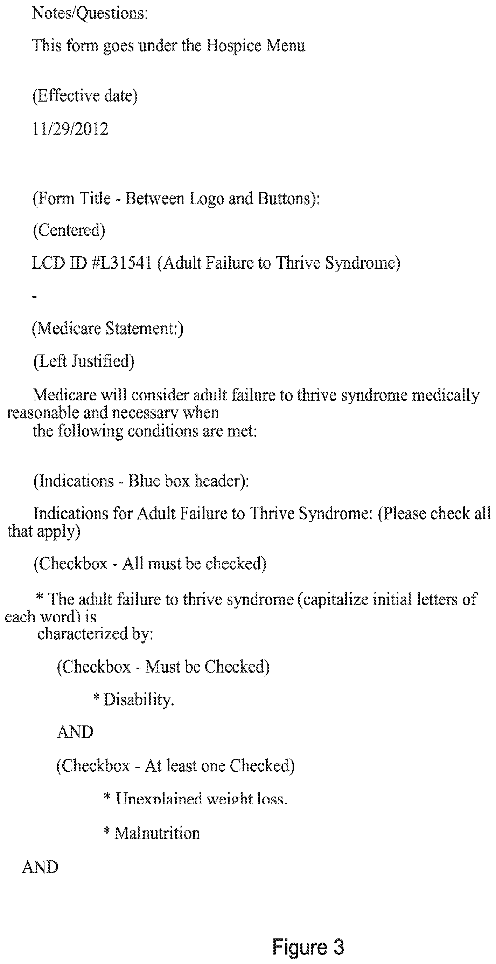

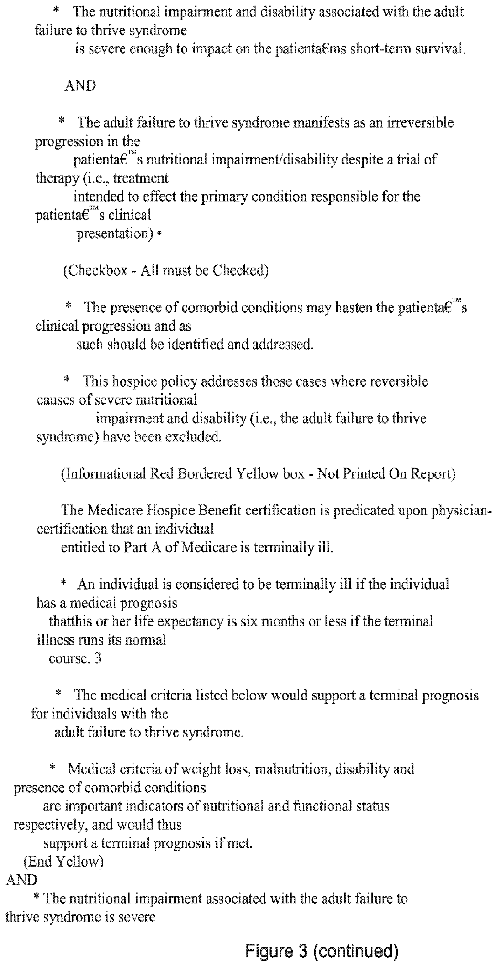

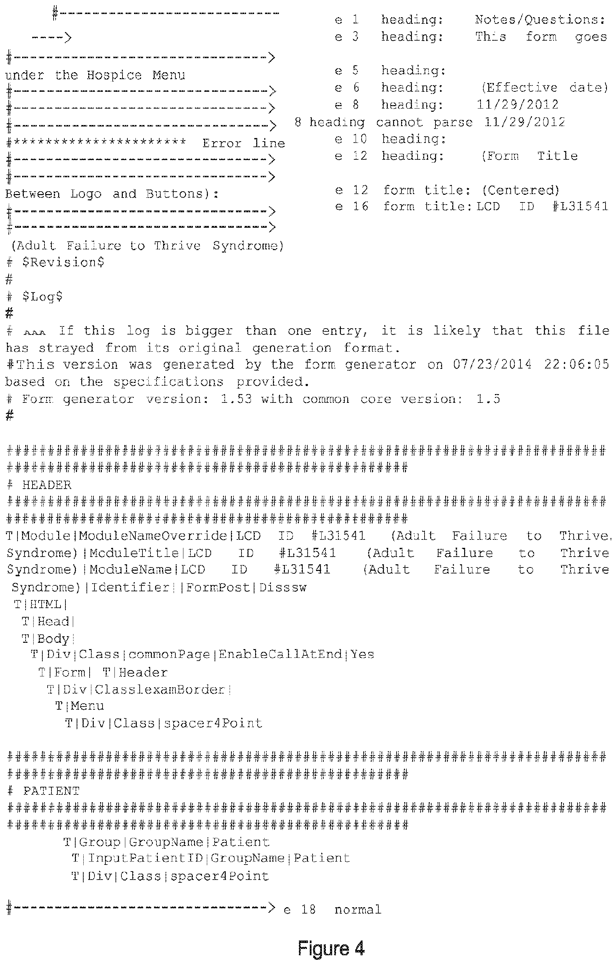

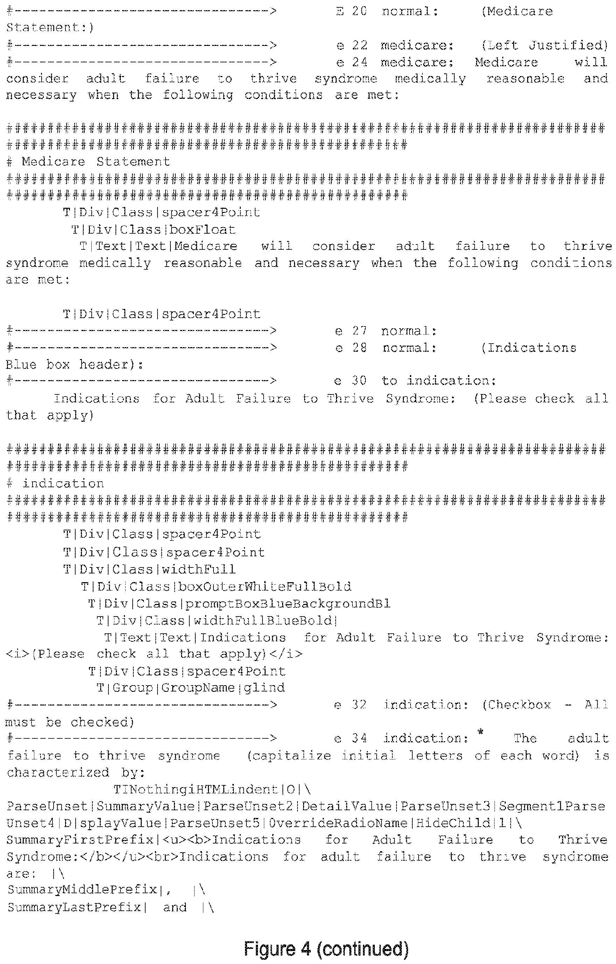

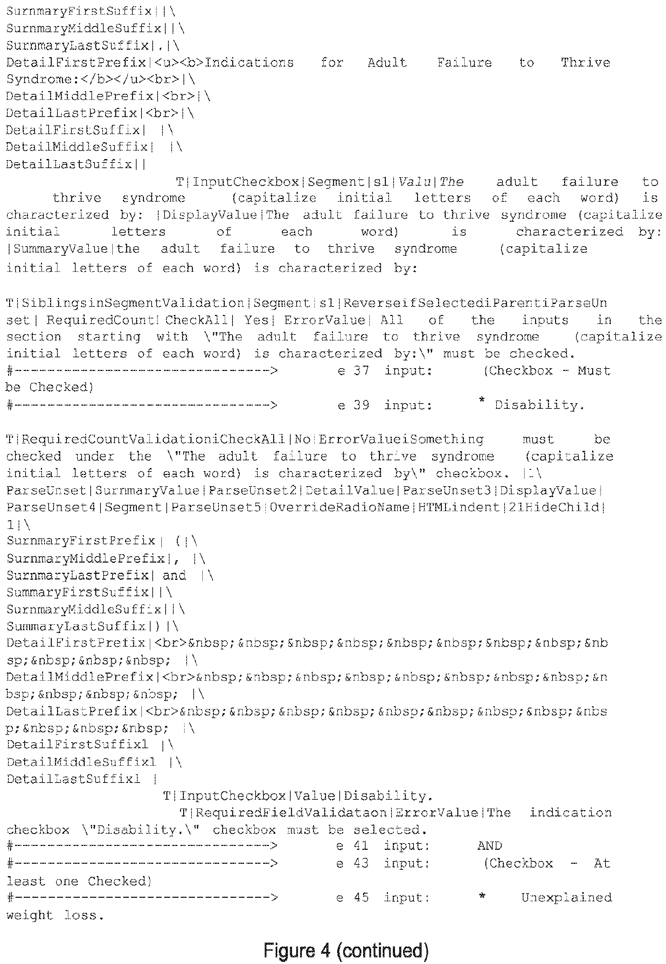

Figure 3 is the scr document and Figure 4 is an excerpt of the T-spec document.

The T-spec document in the T-spec language is then decoded in a PERL module to create the PERL code. When the browser calls the PERL code from the server it is converted to HTML and JavaScript code for display on the screen.

SNOBOL generates errors for code that it does not recognize and those errors are corrected by a programmer and the corrected document is reprocessed through the text manipulation programming language.

Finally the completed code (which represents the form) is loaded onto a server for access by a web browser

The web page is accessed and the form tested. Editors review and test the uploaded web form for correct logic and other errors, making corrections in the underlying code as necessary.

The corrected document is again processed through the text manipulation programming language and the generated code is again loaded on the server

The web page form is then published for access by users

When the form is completed by the user, code embedded within the form determines if the form has been completed. JavaScript determines whether all information has been entered as required.

The JavaScript code will, for example:
Visually display incorrectly selected items (e.g., a radio button was not selected in response to a required questions)
Visually display required items for which no item has been selected
Provide real time notification when selecting specific questions

During data entry, if an item is selected that invalidates a previously selected item a warning message appears on the screen and automatically de-selects the incorrectly selected item. The warning message also explains why the pop up occurred.

Based on the completed form, processing software can generate reports, documents or files with a desired format. The formats comprise:
A natural language narrative format
A detailed/bulleted format
A database compatible format

Since the completed form is in electronic form, processing software can also collect the data recorded in the form, segregates the data into appropriate databases, and conduct analytics on that data.

Since the data entered on the form represents a thorough evaluation of patient health, it can be used to diagnosis a patient's condition. A complete diagnosis can be performed by a physician and certain elements of the diagnosis can be performed by a computer searching through the presented conditions and using logical statements to combine certain presented conditions. Mapping of the collected data to knowledge data bases of various health conditions can assist the physician or the computer with the diagnosis process.

The collected data can also be used to assess comorbidity conditions and assign relative scores to each potential comorbidity condition.

The collected data in the form can be mapped to appropriate codes of the ICD-10 classification system for diseases, signs, symptoms, abnormal findings, complaints, social circumstances, and external causes of injury or diseases, thereby reducing the workload of the physician's staff.

Since the completed form is 100% web native, the form and the entered data can be translated into other languages as well as incorporating web based analytics on all web based transactions and interactions with one or multiple forms.

Figure 5 illustrates the steps required to generate the form as described herein. At a step 10 a document comprising relevant questions and prompts is generated by the subject matter expert, at a step 12 the document is edited for grammatical errors and hints are added at a step 14. At a step 16 the document is converted to a rich text formatted document, then converted to an ".scr" formatted document at a step 18. At a step 20 the document is processed through a text manipulation language to create a T-spec document. PERL/Javascript/HTML/ code is generated at a step 24. The completed form is displayed in a web browser at a step 28, and errors are identified and corrected at a step 32. The corrected/edited form is reprocessed through the text manipulation language (TML) at a step 36. Finally, the completed for is published for use at a step 40.

One advantage of the present invention is the generation of the form programmatically and performance of the subsequent tasks based on the completed form, as described herein. All aspects of the invention can be performed on the same platform; no human intervention is required.

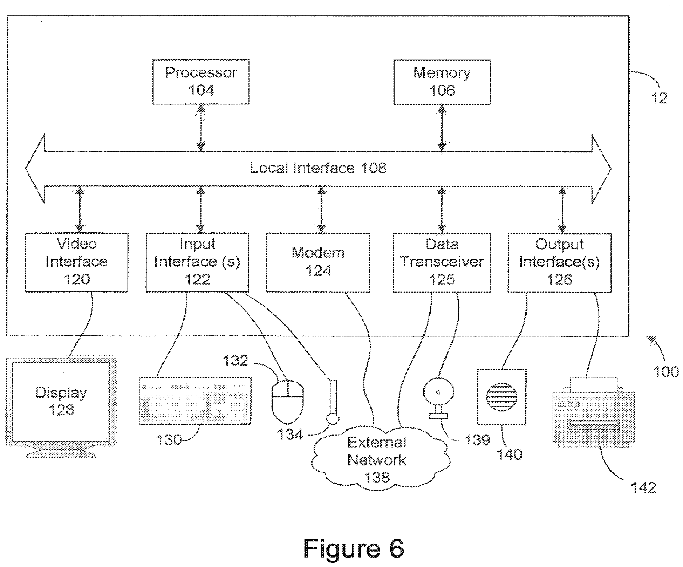

Figure 6 illustrates an exemplary computer system 100 for use in practicing the invention. The system 100 can include multiple local or remotely-located computers and/or processors. The computer system 100 comprises one or more processors 104 for executing instructions in the form of computer code to carry out a specified logic routine that implements the teachings of the present invention. The computer system 100 further comprises a memory 106 for storing data, software, logic routine instructions, computer programs, files, operating system instructions, and the like, as is well known in the art. The memory 106 can comprise several devices, for example, volatile and non-volatile memory components further comprising a random access memory RAM, flash drive memory, a read only memory ROM, hard disks, floppy disks, compact disks including, but not limited to, CD-ROM, DVD-ROM, and CD-RW, tapes, flash drives and/or other memory components. The system 100 further comprises associated drives and players for these memory types.

In a multiple computer embodiment, the processor 104 comprises multiple processors on one or more computer systems linked locally or remotely. According to one embodiment, various tasks associated with the present invention may be segregated so that different tasks can be executed by different computers located locally or remotely from each other.

The processor 104 and the memory 106 are coupled to a local interface 108. The local interface 108 comprises, for example, a data bus with an accompanying control bus, or a network between a processor and/or processors and/or memory or memories. In various embodiments, the computer system 100 further comprises a video interface 120, one or more input interfaces 122, a modem 124 and/or a data transceiver interface device 125. The computer system 100 further comprises an output interface 126 and a display 128. The graphical user interface referred to above may be presented on the display 128.

The system 100 may further comprise several input devices for use by the subject matter expert in creating the initial form and by the user for completing the web based form. These input devices may include, but not limited to, a keyboard 130, a mouse 131, a microphone 132, a digital camera and a scanner (the latter two not shown).

The data transceiver 125 interfaces with system memory 139 where software programs, including software instructions for implementing the various aspects of the present invention are stored.

The modem 124 and/or data receiver 125 can be coupled to an external network 138 enabling the computer system 100 to send and receive data signals, voice signals, video signals and the like via the external network 138 as is well known in the art. The system 100 also comprises output devices coupled to the output interface 126, such as an audio speaker 140, a printer 142, and the like.

As described in detail herein, the present invention offers at least the following advantages over the prior art techniques for generating medical records and the contents of such records.
Data does not include references to personal data of the patient to ensure against HIPAA violations. A medical record number is used in lieu of a patient's name.
Provides risk assessments based on past medical history
Provides co-morbidity scores and quality measurements
Treatment, diagnosis, coding and utilization guidelines are embedded within the platform that
   governs completion of the form to ensure treatments are consistent with payor/insurance carrier requirements for reimbursement. The system identifies non compliant items (e.g., physician did not perform a required test prior to surgery) and informs physician of such non compliance.
Error checking is provided by the platform by comparing the clinical examination and prescribed tests against the compliance guidelines. Any required information (e.g., code, diagnosis) that is absent from the medical record is identified to the physician (e.g., the physician failed to answer a question on the input form)
Audit protection is provided by automatically assigning the correct diagnostic and procedural codes to each diagnosis and procedure and by raising "red flags" when required input information is not entered
Physical examination findings
ICD-9 and ICD-10 diagnostic and CPT and ICD-10PCS procedural codes are entered as the clinical
   examination and tests are performed
Exchanges and analyzes the entered information
Includes a clinical knowledge base
Reduces risks associated with patient care
Serves as an educational resource for the patient and the physician
Includes radiographic documentation
Includes a diagnosis and compliance engine to ensure that patient treatment is in compliance with payor/insurance carrier requirements
For each medical specialty (e.g., pediatrics or orthopedics) and each condition within that specialty, the system includes all applicable codes, differential diagnoses, an overview of the condition, condition symptoms, examination findings, recommended diagnostic tests, recommended acute and chronic therapies and treatments, outcome and educational resources for use by the physician and the patient.
Access to system prompts is driven by answers to previous prompts, i.e., prompts are skipped if not applicable
Mapping of medical information based on algorithms, not databases of information, that lead the user through all aspects of the patient's condition
A drill-down hierarchy unlike the prior art coding systems that are search-based
A system for generating medical documentation and ensuring compliance with applicable guidelines, regulations and laws and ensuring payment for health care services rendered
System prompts and entered information easily translatable to other languages
Medical records data can be mined for further analysis
In one embodiment the system is server based, not database based

The system of the invention further comprises methods and hardware elements to generate and display a dashboard comprising various elements of the patient encounter. The dashboard may include quality-related metrics regarding: (1) situation awareness components (i.e., determining the relevant prompts for a patient encounter based on knowledge of the patient, e.g., do not ask a male patient about a mammogram); (2) ensuring the provider is prompted to action regarding relevant aspects of the patient encounter (e.g., ordering a mammogram for the patient if one has not been performed within the medically-recommended time frame); (3) updating the patient's record with tests to be performed or ordered; (4) disseminating results of the patient encounter to the provider network; (5) updates within the encounter workflow (which varies with measure stereo type) and (6) disseminating work-done notifications, (7) ICD coding based alerts.

Although the present invention has been described as operating with internet access, such is not necessarily required as one embodiment of the invention operates in a standalone mode that does not require internet access, using instead a virtual server (i.e., a server that shares hardware and software resources with an operating system and has a specific URL) with the required code and data elements stored locally. In yet another embodiment the knowledge base can be stored locally with a local processor controlling the content of screens output to the output display device, e.g., prompts for controlling data entry, and receiving and processing the information entered through the input user interface.

The system can also be integrated with an electronic medical records system.

One embodiment of the invention is a system for creating web content consisting of forms using hierarchical formatting that perform real-time error checking, validation and produce standardized reports, that can be easily read by a human or used as an input file for a database, from a simple word processing document by the use of a text manipulation programming language.

Features of the invention/system/platform comprise:
Classification hierarchy decision tree traversal
Text-free entry
Transcription of the path of the tree traversal
Strict adherence to branching factor limit at each tree node constraint propagation classification algorithm breakdown balanced with minimization of clicks to complete traversal
Information keyed by anatomical features
Point & click feature on a cartoon, a line drawing or a diagram when efficient data entry gains are possible
Traversal governed by embedded implications and constraints
   Embedded tests
      Labs
      Imaging
      Stimulation tests
      Device test outputs (pulse oximeter, ECG, EEG, etc.)
   Embedded procedures
      Diagnostic
      Qualifying
      Grading
      Therapeutic
      Palliative indicated
   Embedded treatments
      Options
      Conditional implications
      Previous results
   Embedded codes (unique standardized identifiers for)
      HMS
      Reimbursement
      Prevention
      Reportable disease
      Procedure
      Quality measure
      Incentives
      Compliance
   Embedded guideline compliance
      Record of all requisites
      Conducted actions
      Satisfactions
      Consequent expectations, approvals or permissions Context Sensitive
      Restricted to necessary and sufficient
      Restricted to branching-factor constraint, classification tree traversal path specific (i.e., order of sibling pathways from tree dependent upon context)
      Metadata
         Provider
         Practice
         Scenario
         Acuity
         Pathologic phase
         Epidemiologic features
            Hereditary
            Communicable
            Highly infectious
            Transmission mode
         Recurrence
         Scale/class/index value (e.g., Rankin Scale level) Embedded inclusion criteria
      Negative for requisites
      Prior and Posterior Probability attributes of findings, tests and procedures Embedded exclusion criteria
      Elimination for positive findings
      Prior and Posterior Probability attributes of findings, tests and procedures Consolidated working code such that wrapping technologies (e.g., Google
      Translator) can be employed to extend technical capacity Co-morbidities explicated
   Risk assessment contributors
   Past-history, long and short version
   User Interface
   Immediate visual non-verbal feedback in the form of highlighting to assure rapid success
   Explicit use of the motivating content (coverage determination, guideline, etc.) to govern tree traversal and fabricate output with explicit copy to the highest degree possible
   Abandoned constraint of single screen for an unlimited vertically scrollable form, creating an ongoing perception of rapid progress with feedback
   Back peddling minimized or absent
Multilingual by simply wrapping with Google Translator
Risk determination and quantification
Inherent audit trail provided by tree traversal transcript with zero added effort
Encapsulated and embedded constraints, relationships and compliance criteria propagate by implication and semantics to generate both:
   clinical record
   bulleted audit statement of
      Identification (e.g., which guideline?)
      Justification for purposes of
         Guideline election
         Prequalification
         Coverage determination
         Compliance attestation
         Report generation
Generates structured content, message or standardized input tailored for
   Outcomes analysis
   Results measurement
   Performance monitoring
   Compliance documentation
   Population health management
   Disease and injury tracking both by time, user, location and code
   Interactive decision support
Voice recognition based data entry (similar to constrained input solicited by phone or limited auditory cue technologies)
Maintenance by central updates without disruption of service or use
   Extension by transparent expansion of central services
   Secured and protected by centralized account security and privacy technology including unique user identifiers, authentication and authorization
   Networked with external services and support for content, policy, guidelines, records and status monitoring
   Tailored to site-specific context or customizations for practitioner, practice, network, local region, state or country, etc.
   Generates the data and information necessary to populate dashboards, data warehouses, utility reports, attestation, validation and verification for \ purposes asserted above
   Provides potential for aggregation of alert incidence and nature
   Generate feedback regarding compliance deviations with extended information regarding the scope, frequency distribution and context of deviation and exception, along with factors potentially accounting for variation and repetition risk
   Include formal capture of metadata and conceptual semantic ontology sufficient to ensure adequate support for arbitrary machine query without human mediation necessary
   Capture of clinical information structured with Basic Formal Ontology sufficient to retain the complete semantic meaning of the data such that using the data in the future (population health, outcomes analysis, performance measures, accountable care, process improvement and other purposes mentioned afore, etc.) will not require human mediation
   Risk determination and quantification
   Track progress of specific problem, disease, patient, practitioner, practice, network or region with no loss of generalization (e.g., zip code, altitude, environmental condition, economic strata, population density, race, religion, etc.)
   Extract unanticipated relationships between data elements, adverse events, bio-surveillance markers, epidemiologic indicators, care plan compliance, prognostic reckoning, etc. in real time.
   Achieve the former with capacity to breakdown by age, socioeconomic factors, temporal features, risk categories, genotype, phenotype, incident frequency, etc.
   To prescribe the analytics and analytic variation required for each branch of the classification hierarchy decision tree for each scenario and the process of its traversal as they vary across nodes and branches.
   To prescribe the implications of workflow on the structure of the classification hierarchy decision tree for each scenario, the process of its traversal and ramifications for effective timing in whole or in part.
   Generate alerts regarding discrepancy between actuarial measurements and projected indicator or policy thresholds

The invention may be considered a diagnosis platform as in one embodiment it provides a diagnosis of the patient's presented condition. The diagnosis is based on symptoms and examination findings that are presented during the patient encounter or based on later-conducted tests. The invention or its constituent rules engine suggests/prompts for additional information to ensure the correct diagnosis is reached. The invention further provides ICD10 coding, Medicare coverage determinations and places all the information into properly structured and categorized registries and dashboards (for example, dashboards to measure the quality of the patient encounter).

Although described in the context of a health care environment and an encounter between a physician and a patient, the invention is not limited to health care matters. Instead, the invention is considered a knowledge platform, as created by a subject matter expert, that presents the right information at the right time to a user (using prompts, for example) based on the user's prior inputs. Broadly, the invention documents the user's workflow. The invention populates the educational material as the subject matter expert writes "codes" in a natural language. As the user steps through the system and answers the prompts and questions, the software collects and organizes the responses, performs error checking and prepares the information into a desired format for output. For example, a legal subject matter expert can write a rules engine logic that follows a specific legal guideline or rule of law. The rules engine presents prompts and questions, as previously determined by a subject matter expert, to the user. As the user addresses the prompts and answers questions the collected information leads to a conclusion for the presented legal question.

Note that providing the ICD10 codes for tests, examinations, diagnoses, etc. comprises one element of the invention. The inventive method and system also ensures efficiency and accuracy for Medicare compliance, pre-authorization, diagnosis, treatment pathways, and radiographic findings for complete, accurate, normalized clinical documentation. Teaching of coding, specialty care, and standard guidelines on our real time, error checking, and real time feedback platform at the point of patient care allows for the right information at the right time for standardized patient care and a normalized feedback loop in terms of data feeds to quality dashboards.

In addition to its various advantageous features as described above, the inventive system also serves as a more efficient teaching tool through its embedded knowledge base. One of the inventors, upon receiving the ICD-10 PCS for procedures, a topic about which he knew little, was able to code the procedures in about five minutes-and without attending a course. Similar results can be expected for other knowledge bases embedded within the system. For example, a family doctor or a physician's assistant can learn orthopedics in just a few minutes and an orthopedic surgeon can learn all the pediatrics diagnosis, coding, and treatments quickly by simply following the guided knowledge. Thus the invention can also be useful as a teaching platform for any embedded knowledge.

It should be understood, of course, that the foregoing relates to exemplary embodiments of the invention and that modification may be made without departing from the spirit and the scope of the invention as set forth in the following claims.

## Claims

1. A system for guiding a user's encounter with a patient and for determining a patient medical condition, the system comprising:
a processor (104);
an output display device (128);
an input user interface (122);
the processor (104) configured to send information to the output display device, access the knowledge base, and receive information from the input user interface, wherein the processor is further configured to retrieve a previously generated hierarchical form with an embedded decision tree;
the output display device (128) configured to display the form, and request patient reference information, excluding personal identification information;
the input user interface (122) configured to receive the patient reference information;
the output display device (128) configured to present the form and present prompts for patient medical information, wherein the prompts are generated from the embedded decision tree in the form as the decision tree is traversed with embedded algorithms being executed and from responses to prior prompts, and wherein the patient medical information comprises patient symptoms, patient medical history, and patient vital signs, and further comprising findings from one or more of medical tests, medical procedures, and clinical examinations;
the input user interface (122) for receiving the patient medical information in response to the prompts;
the processor (104) for analyzing the patient medical information and for determining successive prompts;
wherein the prompts and associated patient medical information responsive thereto, form a decision tree with a path through the tree created by each prompt and associated patient medical information;
the path terminating in a patient medical condition further comprising one of a diagnosis, treatment plan, indications and contraindications; and
the output display device (128) for presenting the patient medical condition report.

2. The system of claim 1 wherein information is presented on the output display device (128) as a form for completion by the user and the prompts comprise questions on the form, and wherein the knowledge base comprises a decision engine for use in formulating the successive prompts.

3. The system of claim 1 wherein prompts are presented on the output display device (128) as a web-based form for completion by the user through a browser and the prompts comprise questions on the web-based form.

4. The system of claim 1 wherein the knowledge base comprises standard code identifiers for medical tests, procedures, examinations, and treatments, and wherein the code identifiers are included in the patient medical condition report.

5. The system of claim 1 wherein the processor (104) determines whether the patient medical information entered in response to each prompt is sufficient before presenting a next prompt.

6. The system of claim 1 wherein one or more of the prompts present symptoms for selection by a user.

7. The system of claim 1 wherein the input user interface (122) accepts inputs comprising a gesture, a motion, text entry, key stroke entry, audio entry and radiographic scans.

8. The system of claim 1 wherein one or more of the prompts are in the form of anatomical images, checkboxes, radio buttons, questions with lists of candidate answers, questions eliciting a numerical value answer, point and click responses, and questions soliciting responses in the form of free-text entries.

9. The system of claim 1 wherein under control of the processor (104) and responsive to a user input, the output display device (128) presents medical references and differential diagnoses related to the patient medical condition.

10. A web-based platform for guiding a user's encounter with a patient and for generating a medical record of the encounter, the web-based platform comprising:
a processor (104) in communication with an output display device (128) and an input user interface (122);
the processor (104) for outputting information to the output display device (128) comprising a previously generated hierarchical form with an embedded decision tree for creating successive prompts, and for receiving information from the input user interface (122);
the output display device (128) for presenting successive prompts that request patient medical information, wherein the prompts are responsive to the embedded decision tree being traversed and the execution of embedded algorithms and to prior responses to prior prompts as entered through the input user interface (122); and
the output display device (128) presenting a patient medical condition report based on responses to requests and prompts.

11. A computer program product comprising a non-transitory computer-readable medium having stored thereon computer usable program code, which when executed by a computer, causes the computer to perform the followings steps:
presenting a previously generated hierarchical form with an embedded decision tree to a user, the form comprising one or more prompt fields and one of the one or more prompt fields configured to accept user input;
generating prompt fields from the embedded decision tree by executing embedded algorithms and from responses to prior prompt fields;
receiving patient medical information in response to the prompt fields;
analyzing the patient medical information and determining successive prompt fields;
the prompt fields and responses thereto forming a decision tree with a path through the tree created by each prompt field and each response thereto;
presenting a medical condition report based on a termination of the path; and
assigning an ICD code, if available, to the prompt fields and response thereto after determining that requirements of the ICD code have been satisfied.

12. The computer program product of claim 11 wherein prompt fields are presented on the output display device as a web-based form for completion by the user through a browser and prompt fields comprise questions on the web-based form.

13. The computer program product of claim 11 wherein the knowledge base comprises standard code identifiers for medical tests, procedures, examinations, and treatments, and wherein the code identifiers are included in the patient medical condition report.

14. A method for guiding a user's encounter with a patient and for generating a medical record of the user's encounter, the method comprising:
storing a knowledge base comprising medical information in a decision tree format with paths through the decision tree determined by user responses to prompts for patient medical information, as each prompt is answered a path through the decision tree is created;
presenting a previously generated hierarchical form with an embedded decision tree;
requesting patient identification information;
presenting successive prompts on an output display device, each prompt requesting patient medical information and each prompt responsive to the decision tree and execution of an embedded algorithm and to prior responses to prior prompts as entered through an input user interface;
presenting a medical record on an output display device based on responses to the requests and prompts; and
assigning an ICD code, if available, to the prompts and response thereto after determining that requirements of the ICD code have been satisfied and including ICD codes in the medical report.

15. The method of claim 14 wherein a step of presenting successive prompts comprises presenting successive prompts in a web-based form for completion by the user through a browser, wherein the prompts comprise questions on the web-based form.
